(19) Europäisches Patentamt
European Patent Office
Office européen des brevets

(11) Publication number: 0 043 695 A1

# (12) EUROPEAN PATENT APPLICATION

(21) Application number: 81302989.9

(22) Date of filing: 01.07.81

(51) Int. Cl.³: B 01 J 29/38, C 07 C 2/76, C 07 C 15/00

(30) Priority: 04.07.80 GB 8022081

(43) Date of publication of application: 13.01.82 Bulletin 82/2

(84) Designated Contracting States: BE DE FR GB IT NL SE

(71) Applicant: The British Petroleum Company Limited, Britannic House Moor Lane, London EC2Y 9BU (GB)

(72) Inventor: Davidson, Geoffrey, The British Petroleum Company Ltd. Chertsey Road, Sunbury-on-Thames Middlesex, TW16 7LN (GB)

(74) Representative: Harry, John et al, BP INTERNATIONAL LIMITED Patents and Licensing Division Chertsey Road, Sunbury-on-Thames Middlesex TW16 7LN (GB)

## (54) Activation of gallium ion exchanged aluminosilicate catalyst.

(57) This invention relates to a method of restoring the activity of a gallium/aluminosilicate catalyst system. Such systems get deactivated in use. Such loss of activity in long term use can only be restored by subjecting the system to a sequence of oxidation and reduction treatments. The catalyst systems are useful for hydrocarbon conversion reactions, especially for dehydrocyclodimerisation of paraffins and olefins to aromatics.

EP 0 043 695 A1

# ACTIVATION OF GALLIUM ION EXCHANGED ALUMINOSILICATE CATALYST

The present invention relates to a method of restoring the activity of a gallium ion exchanged zeolite catalyst which has been deactivated during use in a chemical reaction.

Methods of preparing aluminosilicates with a high silica to alumina ratio in which the cations have been exchanged for gallium ions are claimed and described in our British Patent Specification Serial No. 1561590 and in our published copending European Patent Application No. 0002900. These catalysts find use in hydrocarbon conversion reactions, in particular in the dehydrocyclodimerisation of the lower paraffinic hydrocarbons into aromatics. These catalysts maintain a satisfactory level of activity for varying periods, for instance between 60 and 120 hours but thereafter the activity declines to an unsatisfactory level due to, for example, deposition of carbon on the catalyst. Some of the activity of the deactivated catalyst may be restored by burning off the carbon from the catalyst by heating in air at an elevated temperature.

However, after the catalyst has been alternately used and restored in this manner a few times, it begins to suffer from a relatively longer term deactivation and fails to respond to this restoration treatment.

It has now been found that the effects of the long term deactivation may be mitigated by a modified activation procedure.

Accordingly, the present invention is a process for restoring the activity of a catalyst composition comprising an aluminosilicate having a gallium compound deposited thereon and/or an aluminosilicate in which cations have been exchanged with gallium ions, said aluminosilicate

having a silica to alumina ratio of at least 5:1 which process comprises heating the catalyst at an elevated temperature initially in an oxidising atmosphere and subsequently in a reducing atmosphere.

The aluminosilicate having a silica to alumina ratio of at least 5:1 is suitably a zeolite which has a general formula:

$$M_{2/n}O.W_2O_3.yYO_2zH_2O$$

wherein M is a cation which is a positively charged ion selected from a metal ion, an organic ion and a proton of valence n, W is either aluminium or mixtures thereof with gallium, Y is silicon, y is an integer greater than 5 and z is from 0 to 40. The metal ion is preferably an alkali metal ion or an alkaline earth metal ion, preferably sodium or potassium ions. Aluminosilicate zeolites of this type are preferably produced by a process claimed and described in our published copending European Patent Application No. 0002900 in which the zeolite is crystallised from an aqueous solution comprising a mixture of a source of silica, a source of alumina, a source of alkali metal and an organic nitrogen containing base. The organic nitrogen containing base is preferably an alkanolamine.

The cations in the aluminosilicate thus produced are thereafter exchanged with gallium ions by refluxing the crystalline zeolite after water washing and calcination with a solution of a gallium compound. A preferred method of carrying out the gallium ion exchange is claimed and described in our copending published European Patent Application No 0024147.

Where the support has a gallium compound deposited thereon, this may be achieved by impregnation. The impregnation may be achieved by preparing a solution, suitably an aqueous solution, of a gallium compound such as for example gallium nitrate and adding a conventional aluminosilicate to this aqueous solution with thorough stirring to form a paste. The paste is subsequently dried at an elevated temperature in vacuum.

Where the catalyst composition is prepared by using a compound of gallium which ionises in aqueous solution, for example gallium nitrate, it is inevitable that some of the gallium ions will be

exchanged with the cations in the aluminosilicate even if the preparation was directed to impregnation of the aluminosilicate.

Whichever method of catalyst preparation is used, the amount of gallium present in the catalyst compositions may vary for instance between 0.05 and 10 per cent by weight of the total aluminosilicate in the catalyst composition. The gallium exchanged or impregnated zeolite thus obtained may be combined with a porous matrix, e.g. silica or alumina or other inorganic compositions to improve the mechanical strength of the catalyst.

The gallium ion exchanged or gallium compound impregnated aluminosilicate thus produced is the catalyst used in hydrocarbon conversion reactions, especially for the dehydrocyclodimerisation of lower hydrocarbon feedstock. More specifically, the dehydrocyclodimerisation reaction is a catalytic process for the conversion of $C_3$ and $C_4$ gases into a mixture of aromatic compounds which can be used as high octane blending components for gasoline or as feedstock for petrochemicals.

For example, n-butane reacts in the presence of this catalyst at a temperature between 400 and 600°C to form a mixture of aromatic hydrocarbons, including toluene and xylene and generates hydrogen as a by-product. Similarly, propane can be used as a feed to give a similar range of products including benzene. Such dehydrocyclodimerisation reactions using catlysts containing gallium are claimed and described in our British Patent Nos. 1507778 and 1561590.

During the dehydrocyclodimerisation reaction, the catalyst loses some of its activity due to the deposition of carbon. The activity of the catalyst is restored by burning off the carbon deposited on the catalyst. The burn-off is achieved by heating the deactivated catalyst in an oxidising atmosphere containing, for instance, air or oxygen at temperature between 350° and 600°C for between 3 and 18 hours. However, after the loss of catalyst activity due to carbon deposition has been restored a few times by this burn-off procedure, it has been found that the catalyst begins to suffer from a so-called longer term de-activation which

is believed not to be due to carbon deposition. This conclusion stems from the inability of the burn-off procedure to restore the activity of the catalyst deactivated in the longer term. We have now found that the activity of the catalyst affected by the longer term deactivation can be restored by a combination of oxidation and reduction treatments. Thus the catalyst deactivated in the longer term is initially heated at an elevated temperature in an oxidising atmosphere as described above. Thereafter the product of this oxidising treatment is heated at an elevated temperature in a reducing atmosphere. The reducing atmosphere is suitably provided by hydrogen or mixtures thereof with methane and/or ethane. The gases necessary for providing the reducing atmosphere may be derived from the by-products of the dehydrocyclodimerisation reaction. The reduction treatment is suitably carried out at a temperature between 500 and 600°C. The duration of the reduction treatment may be up to 24 hours but is usually not more than 10 hours. In a continuous process for the dehydrocyclodimerisation of alkanes for example, the activity of the deactivated catalyst may be restored by subjecting the catalyst either to a single oxidation treatment followed by a single reduction treatment or, alternatively, the deactivated catalyst may be subjected to a series of sequential runs and oxidative regenerations prior to a single reduction treatment. If the dehydrocyclodimerisation reaction is carried out using the catalyst as a moving bed, the reduction treatment may be achieved using the gaseous by-products of the reaction, which contain hydrogen, as the gas-lift for the moving bed catalyst which has already been subjected to a burn-off treatment.

The present invention is further illustrated with reference to the following Example.

Example

The process for preparing the gallium ion exchanged zeolite was similar to that claimed and described in our copending published European Patent Application No. 0024147, which is as follows:

(a) Zeolite Synthesis

In the synthesis of the zeolite the following reactants were used:

| | |
|---|---|
| Sodium hydroxide | 10.0 g |
| Sodium aluminate | 28.0 g |
| Diethanolamine | 262 g |
| Ludox AS 40 (Registered Trade Mark) | 714 g (40% w/w colloidal silica) |
| Deionised water | 850 g |

Sodium hydroxide and sodium aluminate were dissolved in deionised water (350 g) by warming and stirring for 10 minutes. The solution was then filtered and placed in a 3-litre flask. Diethanolamine was melted and added to this solution and the whole stirred for 10 minutes maintaining the temperature at 40°C. The colloidal silica was then diluted with the remainder of the deionised water (500 g) and then slowly added to the mixture in the flask, over a period of 1 hour. During this addition the temperature was maintained at 40°C and the mixture, which gradually thickened, stirred continuously. Stirring was continued for 0.5 hour after the silica had been added. The mixture was charged to a 3-litre rocking autoclave which was agitated for 4 hours while the temperature was raised to 175°C. The autoclave was then left static at this temperature for 7 days. Thereafter the autoclave was opened and the white crystalline zeolite which had formed was separated from the mother liquor by decantation.

(b) <u>Pre-treatment of Zeolite</u>

The crystalline zeolite was then washed thoroughly first with deionised water and then with a 10% nitric acid solution. Thereafter the acid treated zeolite was washed thoroughly with deionised water to remove any traces of acid. This was then dried in a vacuum oven at 100°C for 16 hours.

The dried zeolite was then calcined in an oven by raising the temperature to 500°C over 4 hours and holding at that temperature for 60 hours.

The calcined zeolite was then refluxed in 1.6 litre of 10% nitric acid for 2.5 hours and then water-washed and dried in a vacuum oven as before.

The acid-washed zeolite was then subjected to ammonium exchange

by refluxing in 1.5 litres of 0.67 molar ammonium nitrate solution for 4 hours. It was then water-washed and dried as previously to give the ammonia exchanged zeolite.

The ammonia exchanged zeolite was recalcined by raising the temperature to 500°C and maintained at that temperature for 16 hours to give the H-zeolite.

(c) Gallium Exchange

The H-zeolite from the recalcination step was placed in 1.65 litre of 0.065 molar solution of gallium nitrate and refluxed for 4 hours. The gallium exchanged material was then water-washed and dried in a vacuum oven as before.

(d) Incorporation of Binder

200 g of gallium exchanged zeolite were mixed with 213 g of Ludox AS 40 (Registered Trade Mark) containing 40% $SiO_2$ and the resulting slurry was dried in a vacuum oven as previously described. The dried product was then broken and sieved to pass 12 to 30 mesh BSS.

(e) The Reaction

A feedstock comprising a mixture of n-butane (83% w/w) and iso-butane (15% w/w) was reacted in a small scale pilot plant at 6.0 LHSV, 6 bar absolute pressure over a 100 ml bed of the above gallium ion exchanged zeolite catalyst maintained at an average bed temperature of 535°C. The catalyst had a total previous life of 23 reaction/oxidative regeneration cycles. The maximum total yield of aromatics from this run was 42% w/w of feed at 6 hours on stream. The activity of the catalyst declined to an aromatic yield of 23% w/w after 30 hours on stream.

After each reaction cycle, the catalyst was subjected to an oxidative burn-off. This was achieved by heating the catalyst from 375 to 550°C in 5% air (equivalent of 1% oxygen) over three hours and then for a further two hours in air alone at 550°C. The burn-off procedure was followed by a purge of the catalyst with nitrogen before the next reaction cycle. After the oxidative regeneration which followed the 27th run the catalyst was additionally treated with hydrogen at 550°C with a GHSV of 1,000 at atmospheric pressure for 12 hours. In a subsequent run with the same feedstock and reaction conditions as before, the peak aromatic yield of 42% w/w of the feed

was repeated but on this occasion the yield of aromatics continued to be over 23% w/w even after the reaction had been on stream for over 69 hours.

## Claims:-

1. A process for restoring the activity of a catalyst composition comprising an aluminosilicate having a gallium compound deposited thereon and/or an aluminosilicate in which cations have been exchanged with gallium ions, said aluminosilicate having a silica to alumina ratio of at least 5:1 for use in a hydrocarbon conversion reaction, which process comprises heating the catalyst at an elevated temperature initially in an oxidising atmosphere and subsequently in a reducing atmosphere.

2. A process according to claim 1 wherein the aluminosilicate having a silica to alumina ratio of at least 5:1 is a zeolite which has a general formula:

$$M_{2/n}O.W_2O_3.yYO_2zH_2O$$

wherein M is a cation which is a positively charged ion selected from a metal ion, an organic ion and a proton of valence n, W is either aluminium or mixtures thereof with gallium, Y is silicon, y is an integer greater than 5 and z is from 0 to 40.

3. A process according to claim 1 or 2 wherein the catalyst is initially heated one or more times in an oxidising atmosphere at a temperature between 350° and 600° and thereafter in a reducing atmosphere.

4. A process according to claim 3 wherein the oxidising atmosphere is provided by air or oxygen.

5. A process according to any one of the preceding claims wherein the catalyst after initial heat-treatment in an oxidising atmosphere is heated one or more times in a reducing atmosphere at a temperature between 500° and 600°C.

0043695

6. A process according to claim 5 wherein the reducing atmosphere is provided by hydrogen, a mixture of hydrogen and methane or ethane, or the by-product gases from the hydrocarbon conversion reaction.

European Patent Office

# EUROPEAN SEARCH REPORT

0043695

Application number
EP 81 30 2989

## DOCUMENTS CONSIDERED TO BE RELEVANT

| Category | Citation of document with indication, where appropriate, of relevant passages | Relevant to claim |
| --- | --- | --- |
| D | GB - A - 1 561 590 (BRITISCH PETROLEUM)<br>* Page 1, line 42 - page 2, line 84; example 8; figure; claims 15,17,20,21 *<br>-- | 1-6 |
| DE | EP - A - 0 024 147 (BRITISCH PETROLEUM) (publ.25-02-1981)<br>* Page 3, line 28 - page 4, line 14;page 4, line 32 - page 5, line 5; claims 1,8,9 *<br>-- | 1-4 |
| A | US - A - 3 450 644 (MOHAMMED,A. LANEWALA et al.) | |
| A | US - A - 4 116 867 (JOHN,W. WARD)<br>---- | |

CLASSIFICATION OF THE APPLICATION (Int Cl.3)

B 01 J 29/38
C 07 C 2/76
C 07 C 15/00

TECHNICAL FIELDS SEARCHED (Int. Cl.3)

B 01 J 29/38

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
| --- | --- | --- |
| The Hague | 09-10-1981 | HOFER |

EPO Form 1503 1 06.78